# EUROPEAN PATENT SPECIFICATION

(11) **EP 1 648 319 B1**
(45) Date of publication and mention of the grant of the patent: **05.08.2009**
(21) Application number: 04722942.2
(22) Date of filing: 24.03.2004
(51) Int. Cl.: A61B 17/70

(54) **INTERLAMINAR VERTEBRAL PROSTHESIS**
ZWISCHENLAMINAE WIRBELSÄULENPROTHESE
PROTHESE VERTEBRALE INTERLAMINAIRE

(30) Priority: 28.03.2003 IT FI20030084
(43) Date of publication of application: 26.04.2006
(73) Proprietor: Smart Hospital S.R.L., 55060 San Marino in Freddana (IT); Cousin Biotech S.A.S., 59017 Wervicq-sud (FR); Petrini, Piero, 06100 Perugia (TO) (IT)
(72) Inventor: PETRINI, Piero, I-06100 Perugia (IT); DENEUVILLIERS, Guy, F-62155 Marlimont (FR)
(74) Representative: Celestino, Marco
(86) International application number: PCT/IT2004/000148
(87) International publication number: WO 2004/084743

(56) References cited:
- EP-A- 1 281 362
- US-A- 5 415 661
- US-A- 5 562 737
- US-A- 5 672 175
- US-A- 5 725 582
- US-A- 6 074 390

## Description

### Technical field

This invention relates to an intervertebral prosthesis capable of redistributing between two adjacent vertebrae the imposed load created by degeneration of the disk located between these vertebrae without immobilizing them, but allowing them the possibility of following normal movements of the spine.

### Background of the invention

Prostheses comprising a part made of deformable material are known. French Patent 2,623,085 in the name of Francis Henri Breard describes a deformable block having two opposite V-shaped ends which can be inserted between the spinous processes of two adjacent vertebrae. The block is secured there through ligatures passing through lateral inclined holes. A prosthesis according to a similar concept is described in European patent publication 0,322,334 A1, inventor Jean-Jacques Bronsard, in which one or more hollow cylindrical elastic cushions are located between the spinous processes of two adjacent vertebrae and secured there through a ligature passing through them. Other interspinous prostheses of various shapes are described in documents FR 2,717,675 and FR 2,775,183 The preamble of claim 1 is based on this document, both in the name of Jean Taylor.

All the abovementioned prostheses are located between the spinous processes and are secured there in order to at least partly absorb the load transmitted between these vertebrae: However, the centre of gravity of the spine is located behind the vertebral body, as a result of which these prostheses - being located between the spinous processes - are somewhat off centre in relation to the centre of gravity. This has the consequence that only a smaller part of that load is in fact taken up by the prosthesis. Furthermore, since for the same reason bending movements of the spine tend to cause the spinous processes to move apart or come together by a relatively large amount, these prostheses can have a tendency to tilt, with the lower part slipping backwards, and therefore losing effectiveness.

### Objects and summary of the invention

The object of the invention is to provide an intervertebral prosthesis which reduces or overcomes at least some of the disadvantages and drawbacks of the known intrervertebral prostheses discussed above.

According to the invention, an intervertebral prosthesis is provided, which includes an elastic body suitable for insertion between two adjacent vertebrae alongside the laminar arch of each vertebra, and means for securing the elastic body to said laminar arches.

In this way the distance between the axis of said elastic body and the centre of gravity of the vertebral bodies is substantially reduced in comparison with the known situations described above. The advantages of locating a damping system which acts interlaminarly are undoubted, as it is unanimously recognized that in degenerative disease of the disk the fulcrum of the functional unit is gradually displaced towards the rear and specifically falls in the interlaminar zone behind the joint surfaces of the vertebrae.

In a preferred embodiment said securing means comprise a plate at each end of the elastic body for anchoring to the laminar arch. Said plates are preferably substantially rigid, preferably more rigid than said elastic body. Each plate has means for connection to the respective vertebra.

In a particularly advantageous embodiment of the invention, said means may include, for each plate, three projections which when the plate is fitted in position face towards the corresponding laminar arch, and in particular: a median projection of a thin shape which can be inserted into the spinal foramen of the vertebra without compressing the spinal cord, and two projections spaced apart laterally for insertion into contact with the posterior surfaces of the laminae forming the laminar arch. The assembly of the plates and the elastic body is held together through at least one ligature passing through holes made in the plates and in the elastic body, holes which, when the assembly is fitted, are in line with each other.

A prosthesis of this kind is especially suitable for the lumbar vertebrae, from L1 to L5, and also between L5 and S1. Because the laminar arch of the vertebrae has a flattened transverse section which is inclined with respect to the axis of the spine, the shapes of the plates generally differ from each other, and can vary according to the pair of vertebrae between L1 and S1 involved.

The elastic body inserted between the plates is of a material which is flexible in all directions, so as to adjust to complex relative movements of the vertebrae. The ligature used to connect the end plates with the elastic body also preferably has some elasticity, and can be provided already attached to a right bevelled needle which is caused to pass through the holes in the plates and the elastic body and secured by rivets once tensioned. The surplus ligature is then cut off and removed.

Preferably, each of said anchoring plates has a groove on the surface in contact with the elastic body into which a tip of divaricator forceps can be inserted in order to move them apart. The technique of fitting the prosthesis according to the invention provides for positioning each plate on the laminar arch of the corresponding vertebra. Then fitting the tips of the divaricator forceps into the grooves in the plates, while these are held in position through the tips of the forceps, the vertebrae are drawn apart placing tension on the ligatures, making it possible for the elastic body to be inserted between the plates, especially from the side, and for the assembly to be subsequently ligated. The technique in fact provides for mono- and/or bilateral access to the intervertebral space and safeguards the supraspinous ligament, with minimum detachment of the ligamentum flavum in the so-called "safety zone" for insertion of the end plates.

Further advantageous embodiments and possible additional features of the prosthesis according to the invention are set forth in the attached claims.

### Brief description of the drawings

The invention will be better understood from the following description and appended drawing which illustrates a non-restrictive embodiment of the invention. In the drawing:
Figures 1 and 2 show views of the assembled prosthesis in elevation, from the side and rear respectively,
Figure 3 shows a side view of the prosthesis fitted in position and in cross-section along the sagittal plane,
Figure 4 shows a rear view along IV-IV in Figure 3,
Figures 5 and 6 show cross-sectional views in plan along V-V and from beneath along VI-VI in Figure 3 respectively,
Figure 7 shows a perspective view of a component of the prosthesis in Figure 1;
Figure 8 shows a perspective view of the prosthesis during a stage of application using divaricator forceps;
Figure 9 shows a lateral view of a further embodiment of the prosthesis according to the invention;
Figures 10 and 11 show end views according to line X-X and XI-XI of Fig.9; and
Figure 12 shows a lateral view of the prosthesis of Figures 9-11 fitted on the spine

### Detailed description of preferred embodiments

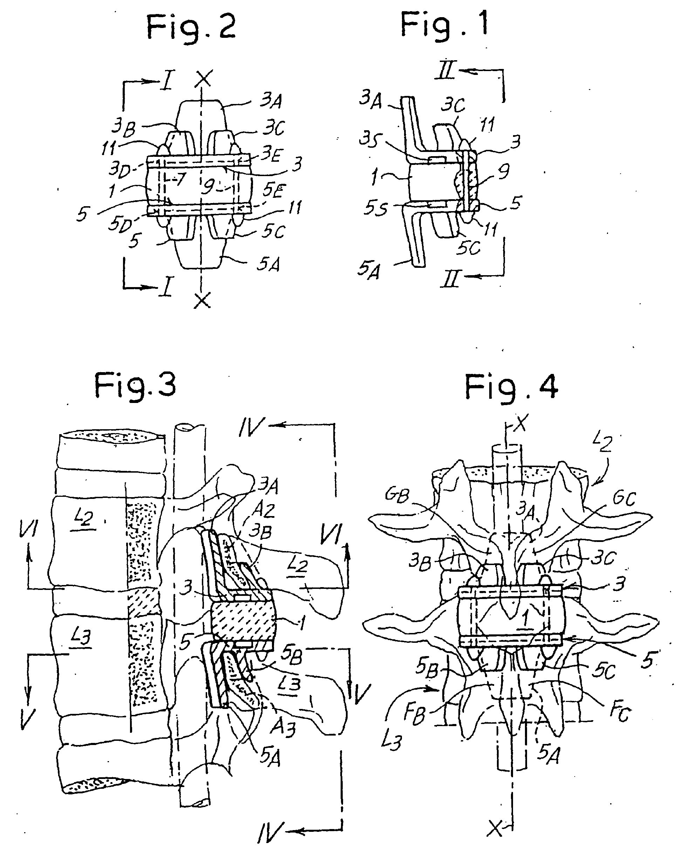

With reference to Figures 1, 2 and 3, a prosthesis according to the invention which has to be inserted between a pair of lumbar vertebrae, for example L2, L3, comprises an elastic body 1 of broadly cylindrical shape located between two end plates 3, 5 each of which can be anchored to the laminar arch of a corresponding vertebra and each of which has a straight groove 3S, 5S on the surface in contact with body 1 orientated at right angles to the sagittal plane along the line X-X (Figure 2). Once fitted the various parts of the prosthesis are stably secured together through two lengths of ligament 7, 9 each of which is caused to pass through corresponding holes 3D, 5D; 3E, 5E; 1 D, 1 E in plates 3, 5 and elastic body 1 respectively, by means of a straight needle which is not shown, and is secured after being tensioned through rivets 11 fitted to the outer surfaces of plates 3, 5.

Body 1 is made of elastic material which is flexible in all directions and may be coated with flexible material (e.g. polyester, etc.) suitable for remaining in contact with human tissues without damaging them or giving rise to rejection reactions.

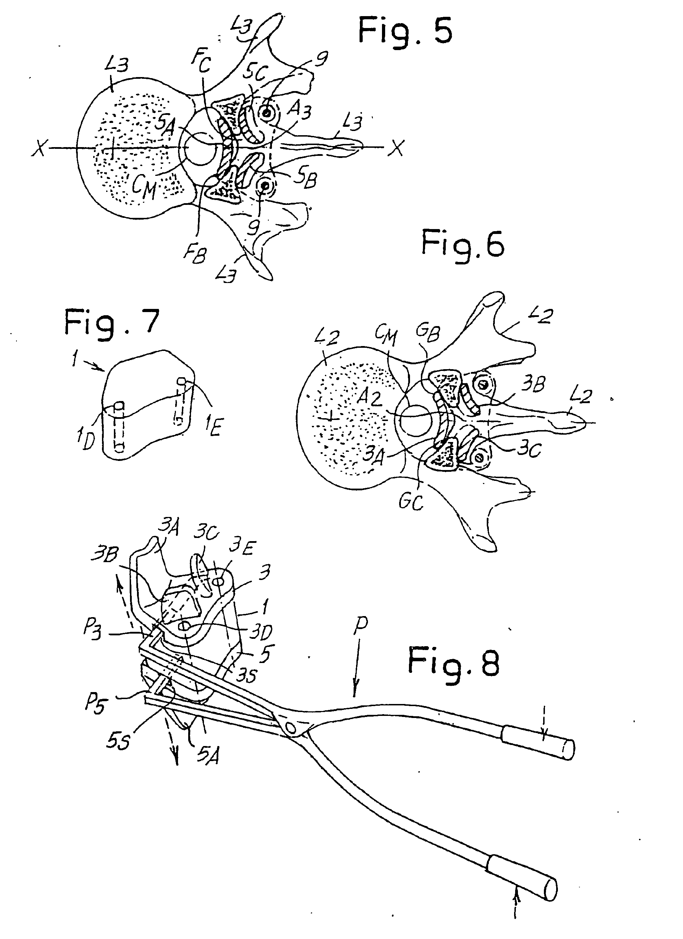

With reference to Figures 1, 2, 3, 4 and 5, upper plate 3 has on its upper surface a central tooth 3A located on the side of the perimeter of the plate facing the spinal foramen CM and a pair of lateral teeth 3B, 3C located symmetrically on opposite sides of the sagittal plane along line X-X, these teeth facing upwards and being slightly inclined with their upper ends towards the left (looking at Figure 3). Having this shape, this plate is dimensioned so that it can be inserted from beneath against the laminar arch A2 (Figure 5) of upper vertebra L2 with central tooth 3A located between the inner central part of laminar arch A2 and spinal foramen CM, and lateral teeth 3B, 3C each in contact with a lateral outer surface (FB, FC) of the corresponding lamina forming said laminar arch A2.

With reference to Figures 1, 2, 3, 4 and 6, lower plate 5 has on its lower surface a central tooth 5A located on the side of the perimeter of the plate facing the spinal foramen CM and a pair of lateral teeth 5B, 5C located symmetrically on opposite sides of the sagittal plane along the line X-X (Figure 5), these teeth facing downwards and being slightly inclined with their lower ends towards the right (looking at Figure 3). Having this shape plate 5 is dimensioned in such a way that it can be inserted downwards against the laminar arch A3 (Figure 6) of lower vertebra L3, with central tooth 5A located between the inner central part of laminar arch A3 and the spinal foramen CM, and lateral teeth 5B, 5C each in contact with a lateral outer surface GB, GC of the corresponding lamina forming said laminar arch A3.

Once plates 3, 5 have been fitted to corresponding vertebrae L2, L3, the terminal tips P3, P5 (Figure 8) of divaricator forceps P are inserted into grooves 3S, 5S and plates 3, 5 together with vertebrae L2, L3 to which they are fitted are moved apart as far as possible. During this stage the divaricator forceps also have the function of holding the plates in position. Thus elastic body 1 can be inserted laterally between plates 3, 5 without having to interrupt the supraspinal ligament, and then ligaments 7, 9 can be passed through appropriate holes 3D, 3E; 5D, 5E in plates 3, 5 and 1D, 1E of body 1 and fixed thereto through rivets 11.

Because the dimensions of the vertebrae vary from one individual to another, and for one individual from one vertebra to another along the length of the spine, these plates are manufactured in various sizes so as to cover a wide range of possibilities of use.

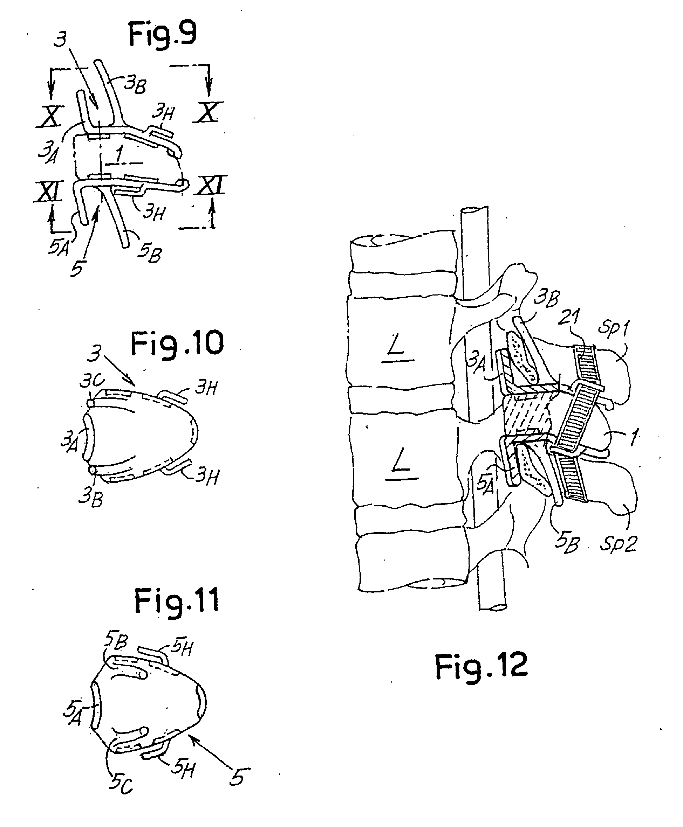

Figures 9-12 show a second embodiment of prosthesis according to the invention. The same reference numbers are used to designate the same or equivalent parts or elements of the prosthesis.

The elastic body 1 has a slightly different shape and is not cylindrical but rather has planar lateral surfaces. Such shape can be used also in the embodiment of the previous figures. The projections 3A, 3B, 3C and 5A, 5B and 5C are shown in a different shape and dimension by way of example. The shape of these portions of the prosthesis can depend upon the location where it has to be applied and/or the morphological characteristics of the patient which has to receive the prosthesis.

The main difference of the prosthesis of Figures 9-12 with respect to the previously described embodiment consists in the presence of an auxiliary ligament 21 which is used to connect the two spinous processes SP1 and SP2 of the adjacent vertebrae designated L in Figure 12, between which the prosthesis is inserted. The ligament 21 embraces the two spinous processes SP1, SP2 and provides an augmentation to the superspinous ligament and a replacement for the interspinous ligament, said auxiliary ligament 21 extending along the same inclined development of the interspinous ligament, i.e. from the apex of the upper spinous process to the base of the lower spinous process.

The ligament 21 is anchored to the two plates 3, 5 by means of suitable connection or anchoring devices. In the example shown in Figures 9-12, each plate 3, 5 is provided with a pair of opposite hooks designated 3H and 5H respectively, on both sides of the plates. The hooks 3H and 5H are oppositely oriented: the hooks 3H are opened backwards, i.e. opposite the spinal foramen, while the hooks 5H are opened towards said spinal foramen.

It will be understood that the drawing only shows an example provided as a practical embodiment of the invention, and the invention may vary in shape and arrangement without thereby going beyond the scope of the concept underlying the invention itself defined in the claims. Any reference numbers included in the appended claims are purely to assist a reading of the claims with reference to the description, and do not restrict the scope of the protection represented by the claims.

## Claims

1. Intervertebral prosthesis comprising an elastic body (1) designed to be inserted between two adjacent vertebrae (L2, L3), wherein each vertebra has a laminar arch (A2, A3), and means (3, 5) for securing the elastic body to said adjacent vertebrae (L2, L3), **characterised in that** said means (3, 5) for securing the elastic body (1) to said adjacent vertebrae (L2, L3) comprises means for engaging said vertebrae between said laminar arches of said adjacent vertebrae (L2, L3), and **in that** said elastic body (1) is inserted between said laminar arches and is of a material which is flexible in all directions.

2. Prosthesis according to Claim 1, **characterized in that** said securing means comprise an anchoring plate (3,5) for each end of the elastic body for anchoring to the corresponding laminar arch (A2, A3) of each of said vertebrae.

3. Prosthesis according to claim 2, **characterized in that** said plate is substantially rigid.

4. Prosthesis according to claim 2 or 3, **characterized in that** each plate has a plurality of projections (3A, 3B, 3C ; 5A, 5B,5C) shaped and arranged for co-acting with the corresponding laminar arch of each vertebra.

5. Prosthesis according to claim 4, **characterized in that** each plate (3,5) has three projections towards the corresponding laminar arch(A2, A3).

6. Prosthesis according to claim 5, **characterized in that** each plate includes a median projection (3A, 5A) designed for insertion into the spinal foramen of the vertebra and two laterally spaced apart lateral projections(3B, 3C ; 5B,5C) designed for insertion in contact with the corresponding outer surfaces(FB, FC ;GB, GC) of the laminae forming the laminar arch (A2; A3).

7. Prosthesis according to claim 6, **characterized in that** said median projection is sufficiently thin so that it can be inserted into the spinal foramen of the vertebra without compressing the spinal cord.

8. Prosthesis according to one or more of the preceding Claims, **characterized by** connection means to connect said securing means (3,5) to said elastic body (1).

9. Prosthesis according to claims 2 and 8, **characterized in that** said connection means include ligatures (7,9), each of which passes through first holes (3D, 3E; 5D, 5E) provided in said plates and corresponding second holes (1D, 1 E) provided in said elastic body, said first holes and said second holes being in line with each other.

10. Prosthesis according to one or more of claims 2 - 7 or 9 **characterized in that** each of said anchoring plates (3, 5) has a groove (3S, 5S) on the surface in contact with the elastic body (1), for the insertion of a corresponding tip (P2, P3) of divaricator forceps (P) in order to separate the vertebrae (L2, L3) between which the prosthesis is to be fitted.

11. Prosthesis according to claim 10, **characterized in that** said grooves (3S, 5S) in the two plates are orientated parallel to each other.

12. Prosthesis according to one or more of the preceding claims, **characterized in that** it further includes an auxiliary ligament 21 for the spinous processes Sp1, Sp2 of the two vertebrae (L2, L3) between which the prosthesis is introduced.

13. Prosthesis according to claims 2 and 12, **characterized in that** at least one of said plates (3,5) includes engaging means 3H,5H for engaging said auxiliary ligament (21).

14. Prosthesis according to claim 13, **characterized in that** each of said plates includes engaging means 3H,5H for engaging said auxiliary ligament (21).

15. Prosthesis according to claim 13 or 14, **characterized in that** said engaging means include at least one lateral hook 3H,5H for at least one of said plates (3,5).

16. Prosthesis according to claim 13, 14 or 15, **characterized in that** said engaging means include two lateral hooks 3H,5H for each of said plates (3,5).

## Patentansprüche

1. Intervertrebrale Prothese, umfassend einen elastischen Körper (1), der zum Einsetzen zwischen zwei benachbarten Wirbeln (L2, L3) bestimmt ist, wobei jeder Wirbel eine Bogenplatte (A2, A3) umfasst, sowie ein Mittel (3, 5) zum Sichern des elastischen Körpers (1) an den benachbarten Wirbeln (L2, L3), **dadurch gekennzeichnet, dass** das Mittel (3, 5) zum Sichern des elastischen Körpers an den benachbarten Wirbeln (L2, L3) ein Mittel zum Einrasten der Wirbel zwischen den Bogenplatten der benachbarten Wirbeln (L2, L3) umfasst, und dass der elastische Körper (1) zwischen den Bogenplatten eingesetzt ist und aus einem Material besteht, das in alle Richtungen flexibel ist.

2. Prothese nach Anspruch 1, **dadurch gekennzeichnet, dass** die Sicherungsmittel eine Verankerungsplatte (3, 5) für jedes Ende des elastischen Körpers zur Verankerung an der entsprechenden Bogenplatte (A2, A3) jedes der Wirbel umfassen.

3. Prothese nach Anspruch 2, **dadurch gekennzeichnet, dass** die Platte im Wesentlichen steif ist.

4. Prothese nach Anspruch 2 oder 3, **dadurch gekennzeichnet, dass** jede Platte mehrere Fortsätze (3A, 3B, 3C; 5A, 5B, 5C) aufweist, die so geformt und angeordnet sind, dass sie mit der entsprechenden Bogenplatte jedes Wirbels zusammenwirken.

5. Prothese nach Anspruch 4, **dadurch gekennzeichnet, dass** jede Platte (3, 5) drei Fortsätze zu der entsprechenden Bogenplatte (A2, A3) aufweist.

6. Prothese nach Anspruch 5, **dadurch gekennzeichnet, dass** jede Platte einen mittleren Fortsatz (3A, 5A) enthält, der zum Einsetzen in das Wirbelloch des Wirbels gestaltet ist, und zwei seitlich beabstandete seitliche Fortsätze (3B, 3C; 5B, 5C), die zum Einsetzen unter Anlage an die entsprechenden Außenfläche (FB, FC; GB, GC) der Laminae, die die Bogenplatte (A2; A3) bilden, bestimmt sind.

7. Prothese nach Anspruch 6, **dadurch gekennzeichnet, dass** der mittlere Fortsatz ausreichend dünn ist, so dass er in das Wirbelloch des Wirbels ohne Zusammenpressen des Rückenmarks eingesetzt werden kann.

8. Prothese nach einem oder mehreren der vorangehenden Ansprüche, **gekennzeichnet durch** Verbindungsmittel zum Verbinden des Sicherungsmittels (3, 5) mit dem elastischen Körper (1).

9. Prothese nach Anspruch 2 und 8, **dadurch gekennzeichnet, dass** die Verbindungsmittel Ligaturen (7, 9) enthalten, von welchen jede durch erste Löcher (3D, 3E; 5D, 5E), die in den Platten bereitgestellt sind, und entsprechende zweite Löcher (1D, 1E), die in dem elastischen Körper bereitgestellt sind, geht, wobei die ersten Löcher und die zweiten Löcher miteinander ausgerichtet sind.

10. Prothese nach einem oder mehreren der Ansprüche 2 bis 7 oder 9, **dadurch gekennzeichnet, dass** jede der Verankerungsplatten (3, 5) eine Rille (3S, 5S) auf der Oberfläche in Kontakt mit dem elastischen Körper (1) zum Einsetzen einer entsprechenden Spitze (P2, P3) einer Divarikatorzange (P) aufweist, um die Wirbel (L2, L3) zu trennen, zwischen welchen die Prothese eingesetzt werden soll.

11. Prothese nach Anspruch 10, **dadurch gekennzeichnet, dass** die Rillen (3S, 5S) in den zwei Platten parallel zueinander ausgerichtet sind.

12. Prothese nach einem oder mehreren der vorangehenden Ansprüche, **dadurch gekennzeichnet, dass** sie des Weiteren ein Hilfsligament 21 für die Dornfortsätze (Sp1, Sp2) der zwei Wirbel (L2, L3) enthält, zwischen welchen die Prothese eingeführt ist.

13. Prothese nach Anspruch 2 und 12, **dadurch gekennzeichnet, dass** mindestens eine der Platten (3, 5) Eingriffsmittel (3H, 5H) zum Befestigen des Hilfsligaments (21) enthält.

14. Prothese nach Anspruch 13, **dadurch gekennzeichnet, dass** jede der Platten Eingriffsmittel (3H, 5H) zum Befestigen des Hilfsligaments (21) enthält.

15. Prothese nach Anspruch 13 oder 14, **dadurch gekennzeichnet, dass** die Eingriffsmittel mindestens einen seitlichen Haken (3H, 5H) für mindestens eine der Platten (3, 5) enthalten.

16. Prothese nach Anspruch 13, 14 oder 15, **dadurch gekennzeichnet, dass** die Eingriffsmittel zwei seitliche Haken (3H, 5H) für jede der Platten (3, 5) enthalten.

## Revendications

1. Prothèse intervertébrale comprenant un corps élastique (1) conçu pour être inséré entre deux vertèbres adjacentes (L2, L3), dans laquelle chaque vertèbre a une courbe laminaire (A2, A3) et des moyens (3, 5) pour fixer le corps élastique auxdites vertèbres adjacentes (L2, L3), **caractérisée en ce que** lesdits moyens (3, 5) pour fixer le corps élastique (1) sur lesdites vertèbres adjacentes (L2, L3) comprennent des moyens pour mettre en prise lesdites vertèbres entre lesdites courbes laminaires desdites vertèbres adjacentes (L2, L3), et **en ce que** ledit corps élastique (1) est inséré entre lesdites courbes laminaires et est réalisé avec un matériau qui est flexible dans toutes les directions.

2. Prothèse selon la revendication 1, **caractérisée en ce que** lesdits moyens de fixation comprennent une plaque d'ancrage (3, 5) pour chaque extrémité du corps élastique pour s'ancrer sur la courbe laminaire (A2, A3) correspondante de chacune desdites vertèbres.

3. Prothèse selon la revendication 2, **caractérisée en ce que** ladite plaque est sensiblement rigide.

4. Prothèse selon la revendication 2 ou 3, **caractérisée en ce que** chaque plaque a une pluralité de saillies (3A, 3B, 3C ; 5A, 5B, 5C) formées et agencées pour co-agir avec la courbe laminaire correspondante de chaque vertèbre.

5. Prothèse selon la revendication 4, **caractérisée en ce que** chaque plaque (3, 5) a trois saillies vers la courbe laminaire (A2, A3) correspondante.

6. Prothèse selon la revendication 5, **caractérisée en ce que** chaque plaque comprend une saillie médiane (3A, 5A) conçue pour l'insertion dans le foramen vertébral et deux saillies latérales (3B, 3C ; 5B, 5C) espacées latéralement, conçues pour l'insertion en contact avec les surfaces externes (FB, FC ; GB, GC) correspondantes des lamina formant la courbe laminaire (A2 ; A3).

7. Prothèse selon la revendication 6, **caractérisée en ce que** la saillie médiane est suffisamment fine de sorte qu'elle peut être insérée dans le foramen vertébral des vertèbres sans comprimer la moelle épinière.

8. Prothèse selon une ou plusieurs des revendications précédentes, **caractérisée par** les moyens de raccordement pour raccorder lesdits moyens de fixation (3, 5) audit corps élastique (1).

9. Prothèse selon les revendications 2 et 8, **caractérisée en ce que** lesdits moyens de raccordement comprennent les ligatures (7, 9), dont chacune passe par les premiers trous (3D, 3E ^{;} 5D, 5E) prévus dans lesdites plaques et les seconds trous (1D, 1E) correspondants prévus dans ledit corps élastique, lesdits premiers trous et lesdits seconds trous étant alignés les uns par rapport aux autres.

10. Prothèse selon une ou plusieurs des revendications 2 à 7 ou 9, **caractérisée en ce que** chacune desdites plaques d'ancrage (3, 5) a une rainure (3S, 5S) sur la surface en contact avec le corps élastique (1), pour l'insertion d'une pointe (P2, P3) correspondante des pinces écarteurs (P) afin de séparer les vertèbres (L2, L3) entre lesquelles la prothèse doit être installée.

11. Prothèse selon la revendication 10, **caractérisée en ce que** lesdites rainures (3S, 5S) dans les deux plaques sont orientées parallèlement l'une par rapport à l'autre.

12. Prothèse selon une ou plusieurs des revendications précédentes, **caractérisée en ce qu'**elle comprend en outre un ligament auxiliaire (21) pour les apophyses épineuses (Sp1, Sp2) des deux vertèbres (L2, L3) entre lesquelles la prothèse est introduite.

13. Prothèse selon les revendications 2 et 12, **caractérisée en ce qu'**au moins l'une desdites plaques (3, 5) comprend des moyens de mise en prise (3H, 5H) pour mettre en prise ledit ligament auxiliaire (21).

14. Prothèse selon la revendication 13, **caractérisée en ce que** chacune desdites plaques comprend des moyens de mise en prise (3H, 5H) pour mettre en prise ledit ligament auxiliaire (21).

15. Prothèse selon la revendication 13 ou 14, **caractérisée en ce que** lesdits moyens de mise en prise comprennent au moins un crochet latéral (3H, 5H) pour au moins l'une desdites plaques (3, 5).

16. Prothèse selon la revendication 13, 14 ou 15, **caractérisée en ce que** lesdits moyens de mise en prise comprennent deux crochets latéraux (3H, 5H) pour chacune desdites plaques (3, 5).
